# EUROPÄISCHE PATENTANMELDUNG

(11) **EP 2 055 769 A2**
(43) Veröffentlichungstag der Anmeldung: **06.05.2009**
(21) Anmeldenummer: 09001430.9
(22) Anmeldetag: 01.04.2006
(51) Int. Cl.: C12M 1/36

(54) **Automatisches Gärsystem und Gärkontrolle**

(30) Priorität: 04.04.2005 DE 102005015530; 21.06.2005 DE 102005028556
(62) Teilanmeldung aus: 06007013.3
(71) Anmelder: Leo Kübler GmbH, 76133 Karlsruhe (DE)
(72) Erfinder: Alber, Bernd, 76227 Karlsruhe (DE)
(74) Vertreter: Lempert, Jost

(57) **Zusammenfassung**

Die Erfindung betrifft ein Verfahren und eine Vorrichtung zum Überwachen eines Gärvorgangs und zur Gärführung. Das Verfahren zeichnet sich dadurch aus, dass die aus dem Behälter entweichende Gasmenge und die durchschnittliche Gärtemperatur bestimmt werden.

Die Vorrichtung weist mindestens eine Messeinrichtung zur Messung der Gasmenge des aus dem Gärgefäß austretenden Gases.

## Beschreibung

Die Erfindung betrifft ein preiswertes Verfahren und eine Vorrichtung zum Überwachen und thermischen Führen eines Gärvorgangs (Gärkontrolle und Gärführung).

Bei der alkoholischen Gärung wird Zucker durch Hefen im Wesentlichen in Alkohol und Kohlendioxyd umgewandelt. Bei der Herstellung von Bier, Spirituosen und Wein ist die Gärung der wichtigste Schritt im Herstellungsprozess. Es ist bei der Herstellung sehr wichtig, diesen Prozess möglichst genau und kontinuierlich zu kontrollieren, um Störungen beim Gären sofort festzustellen, weil sich Störungen negativ auf die Qualität des Getränkes auswirken. Für diese Qualitätskontrolle benötigt man möglichst aktuelle und genaue Daten über die Gärtemperatur, die Zuckerab- bzw. Alkoholzunahme und die Restsüße im Gärgut.

Weit verbreitet ist derzeit noch bei Winzern, Brauern und Brennern die tägliche Messung des Zuckergehalts in Maische und Most per Hand mittels Aräometern und die Verarbeitung dieser Messwerte zu Gärkurven. Dazu werden 200-400 ml Gärgut entnommen, entkohlensäuert und filtriert. Dann wird das Aräometer eingesenkt. Aräometer messen die Dichte des Gärgutes. Dabei werden neben Wasser und Zucker noch bis 15 % Nichtzuckerstoffe (Säuren u.s.w.) miterfasst. Unter Berücksichtigung der Temperatur des Gärgutes erlauben Aräometer eine Abschätzung des Zuckergehaltes der Maische bzw. des Mostes. Dieses Verfahren ist wegen der erforderlichen Vorbehandlung von Most und Maische zeitaufwändig, und auch deshalb nachteilig, weil es in großen Gärtanks von beispielsweise 15.000 Litern erhebliche Dichte- und Temperaturunterschiede gibt, so dass die Messung lediglich einen groben Wert liefert; aber auch deshalb, weil der während der Gärung zunehmende Alkoholgehalt die Messgenauigkeit erheblich beeinträchtigt. Zur Kontrolle des Gärguts wurde bisher der Zuckergehalt meist täglich oder halbtäglich gemessen. Falls es Probleme mit dem Gärprozess gibt, ist dies eine relativ lange Zeitdifferenz, die schnelle Gegenreaktionen bei Gärstörungen einschränken. Die Gärtemperatur wird heute in der Regel mittels eines im Aräometer eingebauten Thermometers gemessen oder dadurch, dass in das Gärbehältnis eine Tauchhülse ragt, in die ein Thermometer eingebaut ist.

Bekannt sind auch Methoden, den Zuckergehalt durch kontinuierliche Dichtemessungen mit in das Gärgut eingebrachten Sensoren zu messen. So ist eine Dichtesonde bekannt, die über die Messung des Absolutdrucks an drei verschiedenen Messpunkten im Gärtank kontinuierlich die Dichte des Gärgutes bestimmt. Die Absolutdrücke ändern sich bei einer Änderung des Füllstands oder bei einer Änderung der Eintauchtiefe im Gärtank. Da die gemessenen Druckdifferenzen zwischen den drei Messpunkten um ein vielfaches kleiner sind als die Änderungen der Absolutdrücke in den gerade genannten Fällen, ist dieses Messverfahren relativ störanfällig und die Bestimmung des Zuckergehalts im Most ist relativ ungenau.

Aus der EP 1 270 716 A1 ist ein Verfahren bekannt, das durch eine Differenzdruckmessung zwischen zwei festgelegten Punkten die Dichte des Gärguts bestimmt und daraus den Zucker- beziehungsweise Alkoholgehalt berechnet. Die Vorrichtung zur Differenzdruckmessung kann fest im Gärtank installiert sein oder auch lösbar. Im ersten Fall sind die Messpunkte fixiert, allerdings müssen dadurch Veränderungen am Tank vorgenommen werden (zum Beispiel durch das Einlassen von zwei Rohren in die Tankwand), was natürlich Kosten verursacht. Im zweiten Fall hat man keine Kosten für die Veränderung des Tanks, allerdings muss bei dieser Methode sichergestellt sein, dass die Messungen stets bei gleichem Höhenabstand der Messpunkte erfolgen. Da Dichtesensoren relativ teuer sind, sind derartige Messvorrichtungen teuer. Außerdem ergeben sich Reinigungsprobleme bei Sensoren, die im Gärgut arbeiten - wie Dichtemesssensoren.

Aus der US 5,204,262 ist ein Verfahren bekannt, das kontinuierlich den Alkoholgehalt im Gärgut über einen diffusionsgesteuerten Ethanolsensor misst. Dieses Verfahren hat den Nachteil, dass der technische Aufwand und die Kosten dafür hoch sind.

Weiterhin ist es schon bekannt, den Gasausstoß durch einen Hitzedrahtanemometer, Balgen- und Trommelgaszähler zu messen.

Aufgabe der Erfindung ist es, ein einfaches, wartungsfreies, preiswertes und genaues Verfahren zur Messung der aus einem Gärtank entweichenden Gasmenge zu schaffen und aufgrund gegebener Zusammenhänge daraus den Zuckerabbau, die Alkoholzunahme und die Restsüße zu ermitteln. Basis ist die Ermittlung des genauen Volumens und des Zuckergehalts des Gärgutes am Gärbeginn.

Erfindungsgemäß wird diese Aufgabe durch ein Verfahren gelöst, mit dem die aus dem Behälter entweichende Gasmenge bestimmt wird. Zur Lösung der Aufgabe ist eine gattungsgemäße Vorrichtung gekennzeichnet durch mindestens eine Messeinrichtung zur Messung der Gasmenge des aus dem Gärgefäß austretenden Gases.

Die Erfassung erfolgt insbesondere "on-line" bzw. in Echtzeit. Um die Messdatenerfassung und die Handhabung der Daten möglichst effizient zu gestalten, sieht eine bevorzugte Ausbildung des erfindungsgemäßen Verfahrens den Einsatz eines Geräts zur elektronischen Datenverarbeitung vor, zum Beispiel einen PC. Auf diese Weise können aus den gemessenen Daten aufgrund bekannter Zusammenhänge, nämlich einer korrigierten Gärgleichung nach Gay-Lussac, bei der berücksichtigt ist, dass Nebenprodukte, wie Glycerin, bis zu 9 % des Alkohols ausmachen können einfach andere Größen, wie zum Beispiel die Zuckerab- oder Alkoholzunahme, berechnet werden und die gemessenen und/oder berechneten Daten gespeichert, ausgedruckt oder grafisch, zum Beispiel als Gärkurven, dargestellt werden.

Durch die Erfindung wird eine genauere Bestimmung der relevanten Größe von Zucker in Most ermöglicht als dies bisher der Fall war.

Die Software der Datenverarbeitung ist so gestaltet, dass Online-Korrekturen einfach vorgenommen werden können. Solche Korrekturen sind zum Beispiel bei Gärstörungen erforderlich, wenn das Gärgut gefiltert werden musste und wenn genaue Messungen gestört und unterbrochen wurden. In einem solchen Fall können die aktuellen Anfangsparameter (Zuckergehalt und Gärgutmenge) neu eingegeben und die Gärkontrolle neu gestartet werden.

Vorteilhafterweise ist vorgesehen, die Messdaten des Sensors von dem Slave per Funk zu einem Master und von diesem per Ethernet an das die Daten verarbeitende Gerät zu übermitteln. Demgemäß sieht die Erfindung bevorzugt, eine Funkeinheit vor, die die ermittelten Daten per Funkt an den Funkempfänger der Auswerteeinheit übermittelt. Dadurch entfällt die aufwändige und teure Verlegung von Kabeln.

Als besonders bevorzugtes Verfahren ist vorgesehen, die Daten von mehreren Sensoren mit einer Auswerteeinheit zu verarbeitet. Es muss dann nicht für jeden Tank eine eigene Auswerteeinheit angeschafft werden. Die Anschaffungskosten pro überwachtem Tank für das System verringern sich dadurch erheblich. Es sollen mit einem einzigen Kontrollgerät bzw. Auswerteeinheit bis etwa zwanzig Gärtanks überwacht werden.

Eine bevorzugte Ausgestaltung sieht dabei vor, dass die einzelnen Messstellen sukzessive abgefragt werden, wobei dies insbesondere zyklisch geschieht, bzw. dass ein durch einen Multiplexer zur Abfrage der Messstellen vorgesehen ist. In Weiterbildung ist dann ein Gerät zur Verarbeitung der gemessenen Daten von mehreren Messstellen vorgesehen.

Bei vielen Gärungen gibt es Störungen, im schlimmsten Fall Gärstopps. Die Behebung von solchen Störungen ist aufwändig. Je schneller die Störung bemerkt und entsprechend reagiert wird, desto geringer ist der finanzielle Nachteil. Deshalb ist es besonders nutzbringend, wenn kontinuierlich die ermittelten Werte automatisch mit vorgegebenen Werten verglichen werden und gegebenenfalls aufgrund von vorher festgelegten Regeln bei Störungen der Gärung oder bei Erreichen vom Gärziel automatisch ein Alarm ausgelöst wird. Demgemäß sieht die Erfindung zumindest eine Alarmeinrichtung vor. Dies kann eine optische, akustische, Wähl- und/oder Sendeeinrichtung sein. Ein Alarm kann so zum Beispiel optisch durch eine Kontrolllampe und/oder akustisch über eine Sirene signalisiert werden. Darüber hinaus sieht die Erfindung auch die Möglichkeit einer telefonischen Benachrichtigung oder eine Benachrichtigung über ein elektronisches Kommunikationsmedium wie zum Beispiel SMS oder E-Mail vor. Hierbei kann selbstverständlich stets der Alarm mehreren Empfängern zugestellt werden. Dies ermöglicht ein schnelles Reagieren selbst zu Zeiten, wenn normalerweise niemand in der Nähe des betreffenden Gärtanks ist, zum Beispiel in der Nacht, am Wochenende oder wenn ein Winzer im Weinberg arbeitet.

Die genaue Messung des Anfangszuckergehalts kann einfach und schnell mit dem neuen Messsystem VINOQUANT 6H von Kübler vorgenommen werden.

Weiter kann vorgesehen sein, dass der Zuckergehalt laufend bestimmt wird.

Erfindungsgemäß werden insbesondere bei folgenden Ereignissen Alarmmeldungen ausgelöst:
- Zu viel Gärgut im Gärbehälter (Gefahr des Überschäumens während der Gärung - unhygienisch; aseptische Wirkung der Sperrflüssigkeit im Gärspund geht verloren). Um diese Gefahr zu beseitigen, beinhaltet das System einen leicht montierbaren Füllstandssensor, der bei ca. 70 % des Behältervolumens am Steigrohr montiert werden sollte (30 % Steigraum).
- Ungeplanter Gärstopp (kein Gärstart innerhalb von 36 Stunden oder Gärstopp vor Gärende).
- Gärende.
- Temperaturschwankungen von mehr als 4°C/Stunde (Gefahr des Hefetodes).
- Temperaturrückgang um mehr als 2°C/Tag am Ende der Gärung (Hefe liegt im Sterben, evtl. bevor durchgegoren ist).
- Zu wenig Sperrflüssigkeit im Gärspund (aseptische Wirkung geht verloren).
- Erreichen eines Gärzieles (z.B. bestimmter Restzuckergehalt zum Stoppen der Gärung ist erreicht).
- Gärführungssolltemperatur nicht erreicht, weil z.B. der Kompressor zu wenig Leistung erbringt.
- Messgerät defekt, Stromausfall.

In weiterer Ausgestaltung ist vorgesehen, die ermittelten Werte automatisch mit vorgegebenen Werten zu vergleichen und gegebenenfalls aufgrund vorher festgelegter Regeln, zum Beispiel bei einem ungewollten Temperaturverlauf, automatisch die Gärung durch äußeres Einwirken auf das Gärgut zu beeinflussen, zum Beispiel durch Heizen oder Kühlen.

Bevorzugt wird erfindungsgemäß die Temperatur des austretenden Gases gemessen. Diese Temperatur ist ein gutes Maß für die Durchschnittstemperatur des Gärguts, weil sie die Temperatur in den verschiedenen Temperaturschichten im Gärtank repräsentiert, im Gegensatz zu der sonst an einem einzigen Ort im Tank (in der Flüssigkeit) gemessenen Temperatur. Letztere kann lokal sehr schwanken und zwar in der Regel von mindestens 5°C und bis zu 10°C. Der Grund sind Hefewolken oder -cluster im Gärgut, in denen eine lokal höhere Gäraktivität und damit auch lokal höhere Temperatur gegeben ist. Daher kann die richtige Temperatur nicht im Tank selbst gemessen werden. Dazu ist der Temperatursensor dicht über dem Stopfen im Loch des Gärtanks oder am Ende des Gärspundes angebracht (in Richtung Gärgut). Dadurch kann sehr schnell die Temperatur des in den Gärbehälter eingefüllten Gärgutes gemessen werden, und zwar vor Gärbeginn. Besonders wichtig ist die neuartige Messung der Temperatur des Gärgutes beim Gärstart und während der Gärung. Diese wichtigen Daten werden durch das Gärsystem automatisch gemessen.

Die erfindungsgemäß gemessene Temperatur ist auch die Basis für die erforderliche Temperaturkorrektur des jeweils gemessenen Gasvolumens und auch die Führung der Gärung durch Erhitzen oder auch Kühlen. Hierzu können vorgegebene Gärkurven herangezogen werden, die ein ideales zeitliches Gärverhalten darstellen. Demgemäß kann aus der Temperatur und dem austretenden Gasvolumen die Stoffmenge des bei einem Gärvorgang entstandenen Kohlendioxyds bestimmt werden. Weiterhin kann vorgesehen sein, dass aus der entweichenden Stoffmenge des Kohlendioxyds für eine Gärkontrolle wichtige Werte bestimmt werden, wie zum Beispiel die Zuckerabnahme und die Alkoholzunahme im Gärgut oder die Restsüße des Gärguts.

Die Temperatur kann man zur Gärführung verwenden. Man kann die Temperatur des Gärguts so von außen beeinflussen, dass sie einerseits so hoch ist, dass die Gärung ablaufen kann und andererseits nicht so hoch ist, dass Aromen aus dem Gärgut heraus mit dem austretenden Gas in die Umwelt abgeführt werden, was zu einem Aromaverlust des Endprodukts führt. Eine solche Gärführung bedeutet, dass die Temperierung an den Verlauf der Gärkurve angepasst wird. Diese ist (relativ) geradlinig. Die Temperatursteuerung im Rahmen der Gärführung erfolgt per Software. Danach ist dazu kein teurer Schaltschrank erforderlich.

Gemäß einer bevorzugten Ausgestaltung des erfindungsgemäßen Verfahrens ist vorgesehen, dass der Zuckergehalt laufend bestimmt wird, wobei insbesondere bei Erreichen einer vorgegebenen Restsüße ein Vorgang ausgeübt wird, wie beispielsweise ein akustisches oder optisches Signal oder aber ein Gärstopp ausgelöst wird.

Eine vorteilhafte Ausgestaltung der erfindungsgemäßen Vorrichtung sieht eine Schnittstelle am die gemessenen Daten verarbeitenden Gerät vor. Die Schnittstelle ermöglicht das Bereitstellen der für eine Gärführung wichtigen Daten, zum Beispiel der Temperatur des Gärguts. Eine an diese Schnittstelle angeschlossene Steuereinheit kann so zum Beispiel durch Wärmen oder Abkühlen des Tanks den Gärvorgang von außen beeinflussen. Erfindungsgemäß kann also die Verarbeitung der Messdaten von mehreren Tanks mit einem Rechner erfolgen.

Das bei der alkoholischen Gärung durch Umwandlung von Zucker mittels Hefen im Wesentlichen in Alkohol entstehende Kohlendioxyd entweicht in üblicher Weise über einen Gärspund aus dem Tank, da sich sonst der Druck in seinem Inneren erhöhen und Schäden verursachen würde.

Die Erfindung sieht insbesondere vor, dass zwischen Gärgefäß und Gärspund eine Volumenstrommessung erfolgt, wobei eine Vorrichtung einen Durchflussmesser insbesondere in einer aus dem Gärgefäß führenden Gas-Rohrleitung, gegebenenfalls zwischen Gärgefäß und Gärspund aufweist.

Diese Durchflussmesseinrichtung kommt insbesondere bei größeren Durchflüssen zum Einsatz.

Eine Weiterbildung dieser Ausgestaltung sieht vor, dass der Durchfluss durch die Spannungsdifferenz zwischen einem stromauf und einem stromab eines Heizers angeordneten Sensor gemessen wird bzw. dass der Durchflussmesser zwei Sensoren und einen Heizer aufweist und jeweils ein Sensor stromauf und stromab des Heizers angeordnet sind und eine Einrichtung zur Messung der Spannungsdifferenz der Sensoren vorgesehen ist.

Durch den Gärspund entweicht Gas aufgrund einer in ihm als Luftabschluss enthaltenen Flüssigkeitsschicht portionsweise aus dem Tank. Das Volumen einer Portion ist dabei immer gleich groß und berechnet sich aus der Hubhöhe und der Geometrie des Gärspunds. Demgemäß sieht die Erfindung in einer bevorzugten Ausführung vor, dass zur Bestimmung entweichender Gasmengen die Hubfrequenz eines beweglichen Deckels eines Gärspunds gemessen wird, wobei eine bevorzugte Ausgestaltung der Vorrichtung gekennzeichnet ist durch eine Messeinrichtung zum Messen der Frequenz eines beweglichen Spunddeckels des Gärspunds. Aus der Hubfrequenz lässt sich unter Berücksichtigung der Geometrie des Gärspunds und Hubhöhe das Gesamtvolumen in einem bestimmten Zeitintervall bestimmen. In Verbindung mit einer Temperaturmessung des austretenden Gases kann aus dem Gasvolumen die Zuckerab- bzw. Alkoholzunahme und die Restsüße im Gärgut berechnet werden.

Die Messung der Hubfrequenz kann auf viele Arten geschehen, jedoch sieht die Erfindung zwei bevorzugte Methoden dazu vor: Die erste ist eine optische Messung, wie eine mit Hilfe einer am Grundkörper des Gärspunds angebrachten Lichtschranke. Die Lichtschranke wird dabei durch eine am beweglichen Deckel des Gärspunds angebrachte "Fahne" unterbrochen wenn der Deckel in seiner unteren Position ist. Bewegt sich der Deckel nach oben, so wird der Raum zwischen der Lichtschranke frei. Alternativ dazu kann die Messung auch elektrisch geschehen, wie über einen Schalter, der am oberen Rand des Gärspundgrundkörpers befestigt ist. Ist der bewegliche Deckel in seiner unteren Position, so wird der Schalter zum Beispiel geschlossen, bewegt sich der Deckel nach oben, so wird der Schalter beispielsweise geöffnet.

Besonders wichtig für das Funktionieren des Gärspunds ist, dass sich immer eine optimale Flüssigkeitsmenge im Gärspund befindet. Die maximale Menge ist durch den am Gärspund vorhandenen Überlauf gegeben, die minimale Flüssigkeitsmenge wird überwacht und es gibt einen Alarm, falls diese unterschritten wird. Bevorzugt erfolgt dies durch ein Bauteil zur Überwachung des minimalen Flüssigkeitsstands im Gärspund, wie einen Reedkontakt. Ist weniger Flüssigkeit im Gärspund wird sich ein am Gärspunddeckel befindlicher Geber (Magnet) einem am Boden des Gärspunds befindlichen Reedsensor stärker annähern und ein Signal bewirken.

Gemäß der Erfindung ist in weiterer Ausgestaltung der Vorrichtung vorgesehen, dass sie im Falle eines automatischen Alarms diesen signalisieren kann. Dabei ist die Alarmierung über ein oder mehrere der nachfolgenden Bauteile vorgesehen:
- optische Alarmeinheiten, wie zum Beispiel Warnleuchten,
- akustisch Alarmeinheiten, wie zum Beispiel Sirenen
- Wähleinheiten, die bei einer oder mehreren vorher festgelegten Telefonnummer(n) anrufen können,
- Sendeeinheiten, die eine oder mehrere SMS, E-Mails oder sonstige elektronische Nachrichten an vorher festgelegte Empfänger versenden können.

Dadurch ist es möglich eine erkannte Gärstörung sehr schnell zu melden, so dass innerhalb kürzester Zeit Gegenmaßnahmen eingeleitet werden können.

Gemäß bevorzugten Ausgestaltungen der Erfindung kann vorgesehen sein, dass der Füllstand im Gärgefäß gemessen wird, und zwar durch einen Füllstandsmesser. Dies ist insbesondere wichtig beim Einsatz von temperaturabhängigen Widerständen als Durchflussmesssensoren, wie sie Gegenstand der Ansprüche 18 und 29 sind, da diese durch Flüssigkeit zerstört werden können. Bei Erreichen eines eingestellten Füllstandes kann ein optisches oder akustisches Signal ausgegeben oder das Zuführen des Gärgutes in das Gärgefäß automatisch gestoppt werden. So sollte beim Beginn der Gärung zwischen der Oberfläche des Gärgutes im Gärgefäß und dem Spundloch ein sogenannter Steigraum von mindestens 1/10 des gesamten Gärbehälterinhaltes vorhanden sein. Erfahrungsgemäß wird dadurch verhindert, dass während der Hochgärphase Gärgut oder Gärschaum durch den Gärspund austreten, mit der Folge einer Beeinträchtigung der aseptischen Wirkung der Sperrflüssigkeit im Gärspund.

Die Erfindung wird im Folgenden anhand von Ausführungsbeispielen näher erläutert, wobei auf die beigefügte Zeichnung Bezug genommen wird. Dabei zeigt:
- Fig. 1: eine schematische Seitenansicht eines Gärtanks mit einem erfindungsgemäß ausgebildeten Gärspund sowie einer Füllstandsüberwachung;
- Fig.2: die schematische Darstellung eines vertikal beweglichen modifizierten Gärspunds zur Durchführung des erfindungsgemäßen Verfahrens;
- Fig.2a: die schematische Darstellung eines vertikal beweglichen modifizierten Kipp- und Gärspunds zur Durchführung des erfindungsgemäßen Verfahrens;
- Fig.3: eine schematische Darstellung einer bevorzugten Ausführungsform der erfindungsgemäßen Vorrichtung;
- Fig.4: eine schematische Gesamtdarstellung einer erfindungsgemäßen Vorrichtung;
- Fig. 5: ein Diagramm zum Gärstopp;
- Fig.6: eine vergrößerte Frontansicht einer ersten Ausführungsform einer erfindungsgemäßen Füllstandsüberwachung;
- Fig.6a: ein Horizontalschnitt durch die Füllstandsüberwachung der Fig. 5;
- Fig.7: eine Frontansicht einer anderen Ausgestaltung der Füllstandsüberwachung;
- Fig.7a: ein Horizontalschnitt durch die Füllstandsüberwachung der Fig. 6;
- Fig.8: eine Frontansicht einer weiteren Ausgestaltung der Füllstandsüberwachung;
- Fig.8a: ein Horizontalschnitt durch die Füllstandsüberwachung der Fig. 7; und
- Fig.9: ein Horizontalschnitt durch eine optische Füllstandsüberwachung.

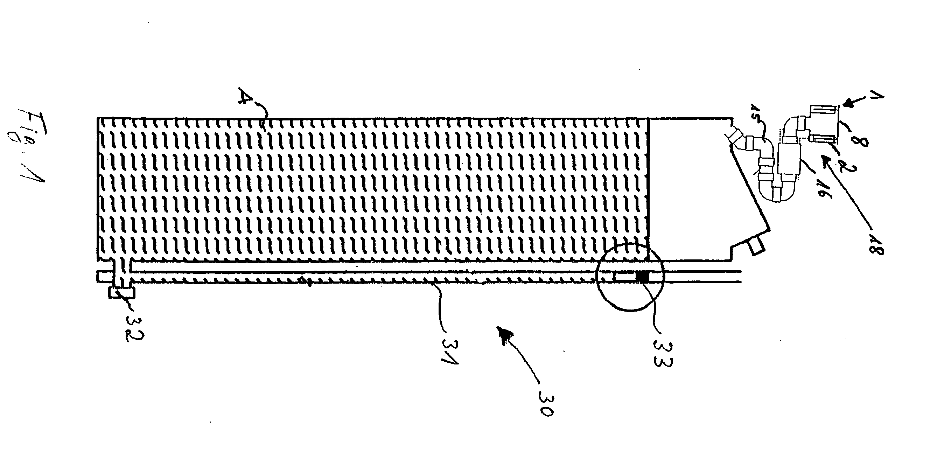

Die Fig. 1 zeigt einen Gärbehälter A, auf dessen Oberseite eine Rohrleitung 15 mit einem Durchflussmesser 16 angeordnet ist. Die Rohrleitung mündet in einen Gärspund ein, der einen doppelzylindrischen Hauptkörper 2 und einen Spunddeckel 8 aufweist, zwischen denen eine weitere Messeinrichtung zur Durchflussmessung 18 angeordnet ist.

Seitlich am Behälter A oder Tank zur Gärung ist ein Füllstandsmesser 30 angeordnet, der über ein Dreiwegehahn 32 mit dem Inneren des Behälters A ein in Fluidverbindung stehendes bzw. bringbares vertikales transparentes Rohr 31 aufweist und in dessen oberen Bereich ein Füllstandssensor 33 im Bereich einer gewünschten Füllstandshöhe angeordnet ist. Die Behältergröße wird so gewählt bzw. der Behälter so gefüllt, dass er nur zu etwa 2/3 gefüllt ist, damit ein Überschäumen durch die Gärung vermieden wird.

Dies wird weiter unten erläutert.

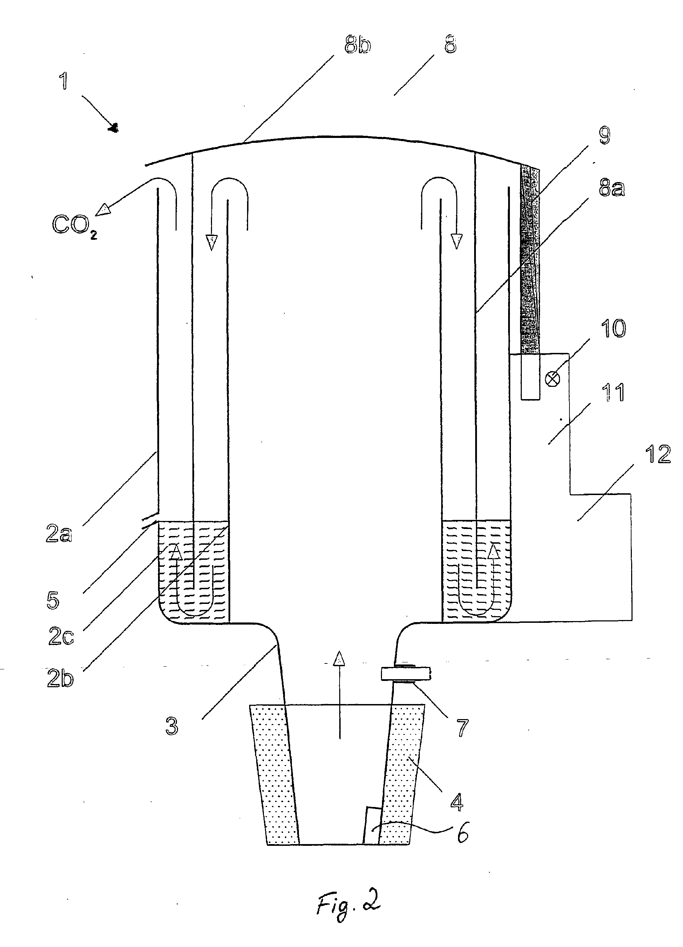

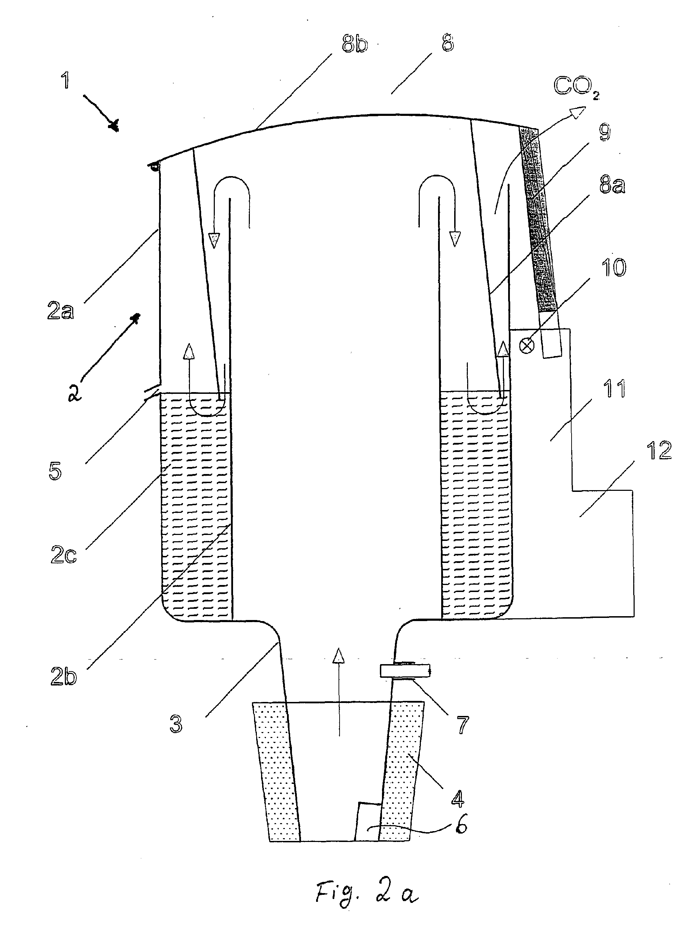

Die Fig. 2 und Fig. 2a zeigen einen zur Durchführung des erfindungsgemäßen Verfahrens modifizierten Gärspund 1. Der erfindungsgemäße Gärspund 1 wird wie jeder andere Gärspund auch mittels eines Stopfens 4 in einer Öffnung im Tank befestigt und erfüllt alle Funktionen, die man von herkömmlichen Gärspunden her kennt. Dies sind unter anderem die Abschirmung des Gärguts nach außen, so dass keine schädlichen Bakterien, Hefen oder Insekten in den Tank gelangen können. Zusätzlich erlaubt ein Gärspund den Austrieb von Gasen aus dem Tankinneren, so dass beim Gären ein Überdruck im Tank verhindert wird.

Der Gärspund 1 weist einen doppelzylindrischen Hauptkörper 2 mit einer äußeren und einer inneren Wandung 2a, 2b auf, der einen verjüngten Einlass 3 aufweist, der von einem elastischen Stopfen 4 umgeben ist, mittels dessen der Gärspund 1 am Auslass eines Gärtanks aufgesetzt werden kann. An der Außenseite der Außenwandung 2a ist ein Auslass 5 zum Auslass überflüssiger Sperrflüssigkeit vorgesehen, die zwischen den beiden Wandungen 2a, 2b des Hauptkörpers 2 eingefüllt ist.

Im Stopfen 4 befindet sich in dem Strömungsweg für das Kohlendioxid im Einlass 3 ein Temperatursensor 6. Unmittelbar oberhalb des Stopfens 4 befindet sich am Einlass 3 ein seitlicher Stutzen 7.

Auf dem Hauptkörper 2 findet sich bei 1. ein vertikal beweglicher, bei 1.a ein um ein horizontales Scharnier kippbarer zylindrischer, auf der Oberseite geschlossener Spunddeckel 8, dessen Zylinderwandung 8a in den Zwischenraum zwischen innerer und äußerer Zylinderwandung 2a, 2b des Hauptkörpers 2 ragt und deren Durchmesser etwa dem Mittelwert der Durchmesser der beiden Zylinderwände 2a, 2b des Hauptkörpers 2 entspricht. An einem Deckteil 8b des Spunddeckels 8 ist seitlich eine Fahne 9 angeordnet, die zwischen ein oder zwei Lichtschranken 10 in einem Mess- und Sendegehäuse 11 ragt, in dem auch der durch den Temperatursensor 6 gemessene Temperaturwert abgenommen wird. Das Mess- und Sendegehäuse 7 schließt im dargestellten Ausführungsbeispiel ein Funkmodul 12 ein, mit dem die erfassten Daten an ein Weiterverarbeitungsgerät, wie ein PC, zur weiteren Verarbeitung übertragen werden können.

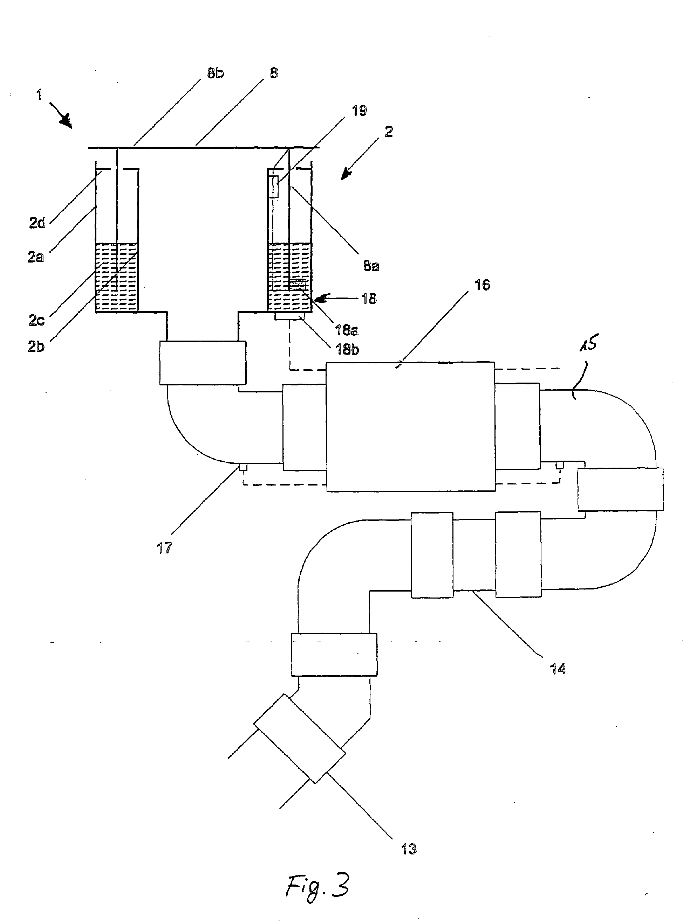

Bevorzugte Ausgestaltungen sind weiterhin in den Fig. 2 und 3 dargestellt. Soweit gleiche Gegenstände dargestellt sind, sind sie mit gleichen Bezugszeichen bezeichnet.

Ein (nicht dargestelltes) Gärgefäß oder ein Tank weist an seiner Oberseite einen Auslassstutzen 13 auf, von dem sich zum Gärspund eine variable, den recht unterschiedlichen Montageplätzen entgegenkommende Rohrleitung 14 erstreckt, in der ein Durchflussmesser 16 angeordnet ist, der insbesondere derart ausgestaltet ist, dass er in Flussrichtung hintereinander angeordnet zwei Sensoren aufweist, zwischen denen ein Heizer vorgesehen ist. Dem Durchflussmesser 16 ist eine Elektronik zugeordnet, die im dargestellten Ausführungsbeispiel um das Rohr 14 und den Durchflussmesser 16 herum angeordnet ist, diese nimmt von den beiden Sensoren Spannungssignale ab. Wenn ein Durchfluss durch die Rohrleitung 14 erfolgt, so ist die Spannungsdifferenz zwischen den beiden Sensoren aufgrund der durch den Heizer bewirkten unterschiedlichen Temperatur (stromauf kühler, stromab wärmer) von der Durchflussgeschwindigkeit und damit der Durchflussmenge abhängig und zu diesen proportional.

In der Leitung 14 ist weiterhin, vorzugsweise stromauf des Durchflussmessers 16, ein Temperatursensor 17 angeordnet, um die Temperatur des aus dem Gärgefäß austretenden Gases zu messen, da diese beispielsweise zur Gewinnung der Durchflussmenge aus dem gemessenen Durchflussvolumen benötigt wird. Grundsätzlich kann der Temperatursensor auch stromab des Durchflussmessers 16 angeordnet sein, da die Aufheizung in diesem aufgrund der geringeren Heizleistung der Durchflussmesssensoren minimal ist und die Messung der Grundtemperatur des aus dem Gärgefäß austretenden Gases kaum beeinflusst.

Im dargestellten Ausführungsbeispiel ist zur Überwachung der Flüssigkeit 2c im Gärspund ein Reedkontakt 18 mit einem am Spunddeckel 8 befestigten Geber 18a in Form eines Magneten und einem Reedsensor 18b am Boden des Gärspundes 1 vorgesehen. Fällt die Sperrflüssigkeit 2c ab, so nähert sich der Geber 18a dem Sensor 18b in stärkerem Maße als bei Normalstand der Sperrflüssigkeit, wodurch ein Signal bewirkt wird. Damit der Geber 18a immer angular in gleicher Position zum Sensor 18b verbleibt, ist eine Verdrehsperre 19 vorgesehen.

Um das Mitreißen und Austreten der Sperrflüssigkeit aus dem Gärspund 1 durch das durch die Sperrflüssigkeit 2c hindurchtretende Gärgas zu verhindern, sind an der Oberseite des Gärspundgefäßes 2 Sperrflüssigkeits-Sperren 2d, 2e, mittels derer die mitgerissene Sperrflüssigkeit zurückgehalten wird und in den Gärspund zurückfließt.

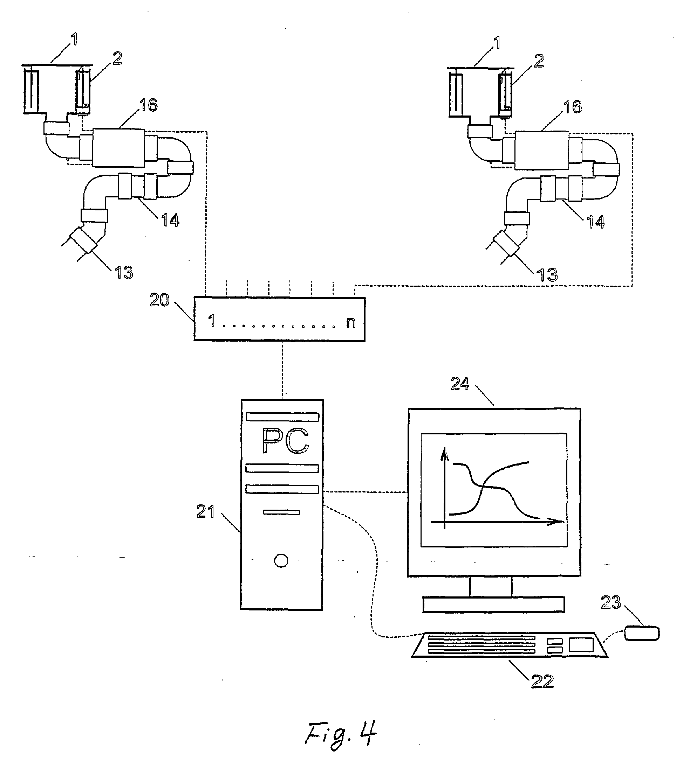

Die Fig. 4 zeigt eine Gesamtdarstellung mit zwei schematisch dargestellten Auslässen und Gärspunden für zwei Gärgefäße. Sämtliche Messstellen sind zu einem Multiplexer 20 geführt und über diesen mit einer Auswerteeinrichtung 21 in Form eines Computers verbunden, der mit Peripheriegeräten, wie einer Eingabetastatur 22 mit Maus 23 und einem Bildschirm 24 versehen ist.

In gleicher Weise können mit einer Auswerteeinrichtung 21 auch weitere Gärtanks, deren Auslässe, Durchflussmesser und erfindungsgemäß ausgestaltete Gärspunde verbunden sein und überwacht werden.

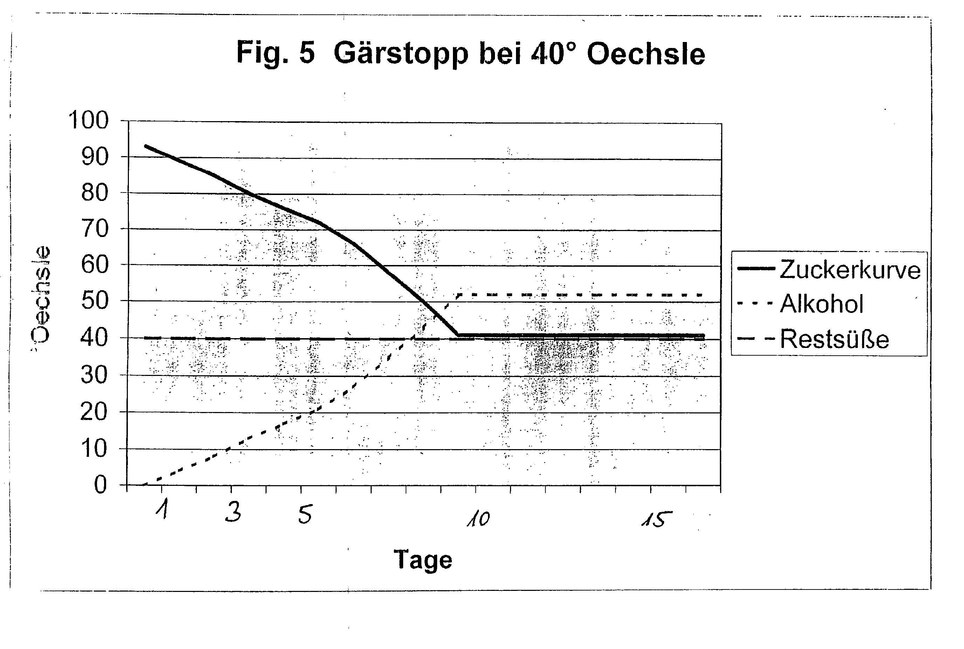

Die Auswerteeinrichtung ist zur Auswertung der gemessenen Daten, insbesondere zur Bestimmung der Zuckerabnahme und des Alkoholgehalts aufgrund des gemessenen CO₂-Durchflusses und der Temperatur ausgebildet. Bei in ihr eingegebener Ausgangssüße lässt sich aufgrund der ermittelten Zuckerabnahme der jeweilige Zuckergehalt bestimmen. Alkohol- und Zuckergehalt sind in der Fig. 5 dargestellt. Es kann in der Verarbeitungseinheit eine gewünschte Restsüße, wie beispielsweise in Fig. 5 von 40° Oechsle eingestellt werden. Wenn der Zuckergehalt diese Restsüße erreicht, kann ein Signal - optisch und/oder akustisch - ausgegeben werden, aufgrund dessen ein Gärstopp eingeleitet werden kann. Auch kann bei Erreichen der gewünschten Restsüße automatisch ein Gärstopp eingeleitet werden, beispielsweise durch Unterbrechung von Erwärmung und/oder durch Kühlung des Gärguts.

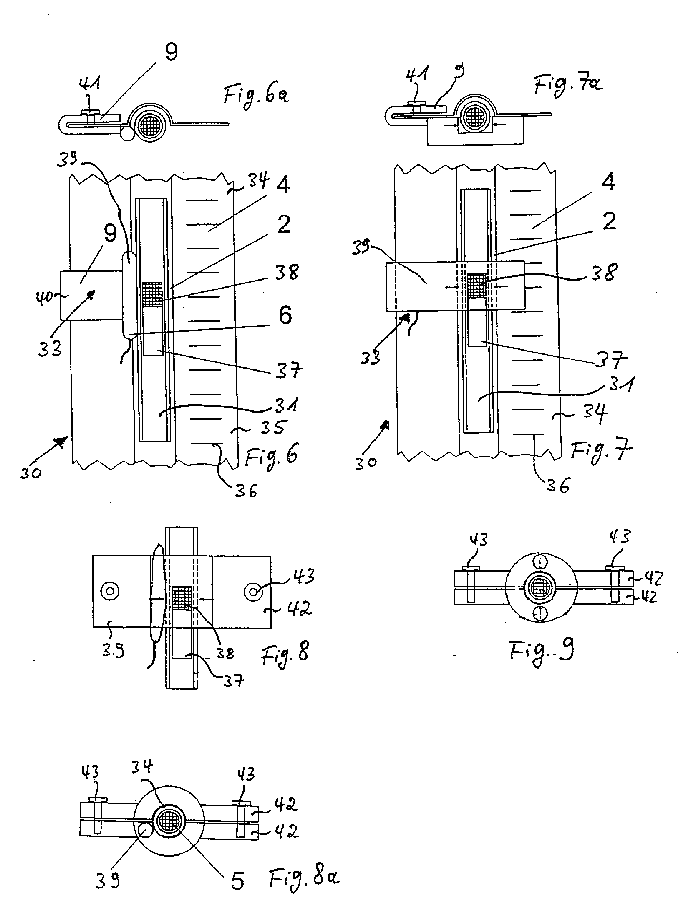

Fig. 6 zeigt in Seitenansicht eine Ausschnittsdarstellung der Füllstandsmessung 30. Diese weist, wie schon gesagt, ein transparentes Rohr 31 sowie einen Füllstandssensor 33 auf. Das Rohr 31 wird durch ein auf der Rückseite desselben zwischen dem Rohr 31 und dem Behälter A angeordnetes Trägerblech 34 getragen, das das Rohr 31 halbkreis- oder Q-förmig umschließt und auf dessen Seitenwangen 35 eine Skala 36 angeordnet ist.

Der Füllstandssensor 33 weist einen im Rohr 31 befindlichen Schwimmkörper 37 auf, der einen Geber 38 trägt, der im dargestellten Ausführungsbeispiel der Fig. 6 und 6a beispielsweise ein Magnet ist. Seitlich des Rohres 33 ist am Trägerblech 34 ein Detektor 39 angeordnet, der im dargestellten Ausführungsbeispiel ein Reeddetektor ist. Der Detektor 39 wird am Trägerblech 34 durch einen Halter 40 gehalten, der im dargestellten Ausführungsbeispiel der Fig. 6 und 6a als im Querschnitt U-förmiger Bügel ausgebildet ist, der eine Klemmschraube 41 trägt, mit der der Detektor 39 auf der gewünschten Füllstandshöhe am Träger 34 festklemmbar ist.

Beim Ausführungsbeispiel der Fig. 7 und 7a ist der vom Schwimmkörper 37 getragene Geber 38 ein undurchsichtiger Körper. Der Detektor 39 ist als optischer Detektor ausgebildet, der, wie in Fig. 7a dargestellt ist, das Rohr und damit den Geber 38 U-förmig umgreift und eine Lichtquelle sowie einen optischen Empfänger aufweist (nicht dargestellt), so dass bei Erreichen des Füllstandes, in dem der Füllstandssensor 30 angeordnet ist, der Lichtweg von der Lichtquelle zum Lichtempfänger durch den Geber 38 unterbrochen wird. Der Füllstandssensor ist bei dieser Ausgestaltung der Fig. 7 und 7a in gleicher Weise am Träger 34 befestigt, wie dies unter Bezugnahme auf die Fig. 6 und 6a beschrieben wurde.

Bei der Ausgestaltung der Fig. 8 wird ein Detektor, der ebenfalls, wie bei den Fig. 6 und 6a ein magnetischer Detektor in Form eines Reedkontaktes ist, durch zwei das Rohr 34 jeweils halbkreisförmig umgebende Platten 42 gehalten, die durch zwei beidseits des Rohres 34 angeordnete Schrauben 43 mit dem Rohr verspannbar sind.

Bei der Ausgestaltung der Fig. 9 ist der Halter in gleicher Weise ausgebildet wie bei der Ausgestaltung der Fig. 8 und 8a. Die Detektoreinrichtung 39 ist wiederum als optische Detektoreinrichtung mit diagonal gegenüberliegenden, jeweils in einer in der halbkreisförmigen Ausbildung einer der Platten 42 angeordneten Lichtsender und Lichtempfänger ausgebildet, wobei der Lichtweg zwischen diesen wiederum durch den in Form eines undurchsichtigen Körpers ausgebildeten Gebers 38 bei Erreichen des eingestellten Füllstandes unterbrochen wird.

Die Detektoren 39 können über eine Leitung 44 mit der Auswerteeinrichtung 21 (Fig. 4) verbunden sein. Bei Erreichen des eingestellten und gewünschten Füllstandes kann ein Signal, wie ein optisches oder akustisches Signal, ausgegeben werden. Durch die Auswerteeinrichtung kann auch ein Ventil in einer Zulaufleitung zum Behälter A (nicht dargestellt) automatisch geschlossen werden.

### Bezugszeichenliste

- 1: Gärspund
- 2: doppelzylindrischer Hauptkörper
- 2a: äußere Wandung
- 2b: innere Wandung
- 2c: Flüssigkeit
- 2d, 2e: Sperrflüssigkeits-Sperren
- 3: Einlass
- 4: Stopfen
- 6: Temperatursensor
- 7: seitlicher Stutzen
- 8: Spunddeckel
- 8b: Deckteil
- 9: Fahne
- 10: Lichtschranken
- 11: Sendegehäuse
- 12: Funkmodul
- 13: Auslassstutzen
- 14: Rohrleitung
- 15: Rohrleitung
- 16: Durchflussmesser
- 17: Temperatursensor

- 18: Reedkontakt
- 18a: Geber
- 18b: Reedsensor
- 19: Verdrehsperre
- 20: Multiplexer
- 21: Auswerteeinrichtung
- 22: Eingabetastatur
- 23: Maus
- 24: Bildschirm
- 30: Füllstandsmesser
- 31: transparentes Rohr
- 32: Dreiwegehahn
- 33: Füllstandssensor
- 34: Trägerblech
- 34: Rohr
- 35: Seitenwangen
- 36: Skala
- 37: Schwimmkörper
- 38: Geber
- 39: Detektor
- 40: Halter
- 41: Klemmschraube
- 42: Platten
- 43: Schrauben
- 44: Leitung
- A: Gärbehälter

## Patentansprüche

1. Verfahren zum Überwachen eines Gärvorgangs in einem Gärgefäß, **dadurch gekennzeichnet, dass** die aus dem Behälter entweichende Gasmenge bestimmt wird.

2. Verfahren nach Anspruch 1, **dadurch gekennzeichnet, dass** die ermittelten Werte automatisch mit vorgegebenen Werten verglichen werden und gegebenenfalls aufgrund vorher festgelegter Regeln automatisch die Gärung durch äußeres Einwirken auf das Gärgut, wie Heizen oder Kühlen, beeinflusst wird.

3. Verfahren nach einem der Ansprüche 1 oder 2, **dadurch gekennzeichnet, dass** die Übertragung der ermittelten Werte drahtlos an eine Auswerteeinheit übermittelt werden.

4. Verfahren nach einem der Ansprüche 1 bis 3, **dadurch gekennzeichnet, dass** die ermittelten Werte elektronisch verarbeitet, gespeichert, ausgedruckt und/oder graphisch aufgetragen werden.

5. Verfahren nach einem der Ansprüche 1 bis 4, **dadurch gekennzeichnet, dass** die ermittelten Werte automatisch mit vorgegebenen Werten verglichen werden und gegebenenfalls aufgrund vorher festgelegter Regeln, insbesondere bei Gärstörungen, automatisch ein Alarm ausgelöst wird.

6. Verfahren nach einem der Ansprüche 1 bis 5, **dadurch gekennzeichnet, dass** die Daten mehrerer Sensoren mit einer Auswerteeinheit verarbeitet werden.

7. Verfahren nach einem der vorangehenden Ansprüche, **dadurch gekennzeichnet, dass** die Gasmengenbestimmung durch eine Volumenstrommessung erfolgt.

8. Verfahren nach einem der Ansprüche 1 bis 7, **dadurch gekennzeichnet, dass** die Temperatur des austretenden Gases gemessen wird.

9. Vorrichtung zum Überwachen eines Gärvorgangs in einem Gärgefäß, **gekennzeichnet durch** mindestens eine Messeinrichtung zur Messung der Gasmenge des aus dem Gärgefäß austretenden Gases.

10. Vorrichtung nach Anspruch 9, **gekennzeichnet durch** eine elektronische Auswerteeinheit zur elektronischen Verarbeitung, Speicherung und Ausgabe der ermittelten Werte.

11. Vorrichtung nach Anspruch 10, **gekennzeichnet durch** eine Einrichtung (21) zum automatischen Vergleichen der ermittelten Werte mit vorgegebenen Werten und zum automatischen Beeinflussen der Gärung **durch** äußeres Einwirken auf das Gärgut, wie Heizen oder Kühlen, aufgrund vorher festgelegter Regeln.

12. Vorrichtung nach einem der Ansprüche 9 oder 10, **gekennzeichnet durch** einen Durchflussmesser in einer aus dem Gärgefäß herausführenden Gas-Rohrleitung (14, 15).

13. Vorrichtung nach einem der Ansprüche 9 bis 12, **gekennzeichnet durch** einen Temperaturfühler zur Messung der Temperatur des austretenden Gases.

14. Vorrichtung nach einem der Ansprüche 9 bis 13, **gekennzeichnet durch** einen Multiplexer zur Abfrage der Messstellen.

15. Vorrichtung nach einem der Ansprüche 9 bis 14, **gekennzeichnet durch** eine Schnittstelle zur Bereitstellung von für die Gärführung benötigten Daten.
